(19) Europäisches Patentamt
European Patent Office
Office européen des brevets

(11) **EP 1 310 785 B1**

(12) **EUROPÄISCHE PATENTSCHRIFT**

(45) Veröffentlichungstag und Bekanntmachung des Hinweises auf die Patenterteilung:
**27.02.2013 Patentblatt 2013/09**

(51) Int Cl.:
*A61B 6/03* (2006.01)  *G01N 21/64* (2006.01)
*G01N 23/04* (2006.01)

(21) Anmeldenummer: **02102555.6**

(22) Anmeldetag: **11.11.2002**

(54) **Fluoroskopisches Computertomographie-Verfahren, Computertomograph und Computerprogramm zur Verarbeitung der Messwerte eines Computertomographen**

Fluoroscopic computer-tomography method, computer-tomograph, and computer program for processing the measurement values of a computer tomograph

Procédé de tomographie de fluorescence assistée par ordinateur, tomographe assisté par ordinateur et logiciel pour le traitement des données mesurées par un tomographe assisté par ordinateur

(84) Benannte Vertragsstaaten:
**DE FR GB**

(30) Priorität: **13.11.2001 DE 10155590**

(43) Veröffentlichungstag der Anmeldung:
**14.05.2003 Patentblatt 2003/20**

(73) Patentinhaber:
• **Philips Corporate Intellectual Property GmbH
20099 Hamburg (DE)**
Benannte Vertragsstaaten:
**DE**
• **Koninklijke Philips Electronics N.V.
5621 BA Eindhoven (NL)**
Benannte Vertragsstaaten:
**FR GB**

(72) Erfinder:
• **Grass, Michael, Dr.
c/o Philips Corporate
52088, Aachen (DE)**
• **Köhler, Thomas, Dr.
c/o Philips Corporate
52088, Aachen (DE)**

(74) Vertreter: **Volmer, Georg et al
Philips Intellectual Property & Standards GmbH
Postfach 50 04 42
52088 Aachen (DE)**

(56) Entgegenhaltungen:
**EP-A- 0 919 954     EP-A- 0 989 520
EP-A- 0 990 892**

**Beschreibung**

Die Erfindung betrifft ein fluoroskopisches Computertomographie-Verfahren zur Durchführung eines solchen Verfahrens und ein Computerprogramm für eine derartige Bildverarbeitungseinrichtung.

Aus der EP 948930 ist ein Fluoroskopiesystem bekannt, das gleichzeitig auch als CT-System betrieben werden kann (CT = Computertomographie), um ein dreidimensionales CT-Bild zu erzeugen ("volume CT"). Das Fluoroskopiesystem umfaßt eine Gantry mit einer Strahlenquelle und einem Flachdetektor, die innerhalb eines Zeitraumes zwischen 5 und 90 Sekunden einmal um eine Rotationsachse rotieren und dabei 300 bis 500 zweidimensionale Durchleuchtungsbilder erzeugen, aus denen ein dreidimensionales CT-Bild rekonstruiert werden kann.

Aus der EP 919 954 ist ein Verfahren zur fortlaufenden Rekonstruktion von CT-Bildern bekannt. Die europäische Patentanmeldung EP 989520 offenbart ein Computertomographie-Verfahren mit kegelförmigem Strahlenbündel.

Demgegenüber wird im Sinne der Erfindung unter fluoroskopischer Computertomographie ein Verfahren verstanden, bei dem nicht nur ein einzelnes dreidimensionales CT-Bild, sondern fortlaufend CT-Bilder erstellt werden, die das Untersuchungsobjekt dreidimensional in aufeinanderfolgenden Abbildungszeitpunkten darstellen, wobei der zeitliche Abstand zwischen zwei aufeinanderfolgenden Zeitpunkten kleiner sein soll als der für eine Rotation des Untersuchungsobjektes (bei industriellen Objekten) oder Strahlenquelle (bei medizinischen Untersuchungen) um die Rotationsachse benötigte Zeitraum.

Ein solches Verfahren wird beispielsweise bei der CT-geführten Biopsie benötigt, wobei eine Biopsienadel in ein Untersuchungsobjekt eingeführt wird und der Vorschub der Biopsienadel fortlaufend anhand einer Folge von dreidimensionalen CT-Bildern kontrolliert wird. Bei solchen Untersuchungen ist es wichtig, daß die CT-Bilder einen möglichst breiten Bereich des Untersuchungsobjekts wiedergeben und möglichst wenig Bewegungsartefakte aufweisen. Die Bewegungsartefakte werden dadurch hervorgerufen, daß zur Rekonstruktion Meßwerte herangezogen werden, die zu unterschiedlichen Zeitpunkten akquiriert worden sind.

Aufgabe der Erfindung ist es daher, ein Computertomographieverfahren zu schaffen, das diesen Forderungen genügt. Diese Aufgabe wird erfindungsgemäß gelöst durch ein fluoroskopisches Computertomographie-Verfahren nach Anspruch 1 mit den Schritten:

- Fortlaufende Durchstrahlung eines Untersuchungsobjekts mit einem von einer Strahlenquelle emittierten kegelförmigen Strahlenbündel, wobei das Untersuchungsobjekt oder die Strahlenquelle um eine Rotationsachse rotieren,
- fortlaufende Akquisition von Meßwerten, die von der Intensität in dem Strahlenbündel jenseits des Untersuchungsobjektes abhängen, mit einer Detektoreinheit,
- fortlaufende Rekonstruktion von CT-Bildern, die das Untersuchungsobjekt in Abbildungszeitpunkten darstellen, deren zeitlicher Abstand voneinander kürzer ist als der für eine Rotation des Untersuchungsobjektes oder der Strahlenquelle um die Rotationsachse benötigte Zeitraum, wobei zu rekonstruierende Voxel solche Voxel beinhalten, die nicht ständig von Strahlung getroffen werden, wobei die Rekonstruktion folgende Schritte umfasst:

  a) Rebinning der Meßwerte zu einer Anzahl von Gruppen, wobei jede Gruppe die Messwerte von bei der Durchstrahlung erzeugten Strahlenfächern umfasst, die in zueinander und zur Rotationsachse parallelen Ebenen liegen,
  b) eindimensionale Filterung der durch das Rebinning erzeugten Daten einer jeden Gruppe zur Erzeugung gefilterter Daten,
  c) Rückprojektion der gefilterten Daten von mehreren Gruppen, wobei für jedes zu rekonstruierende Voxel gefilterte Daten aus den letzten vor dem jeweiligen Abbildungszeitpunkt erzeugten Strahlenfächern herangezogen werden, die das betreffende Voxel aus einem Gesamt-Projektionswinkelbereich von gerade 180° durchstrahlt haben.

Ein Computertomographieverfahren mit den Schritten a) und b) ist aus einem Aufsatz von Grass et al in Phys. med. Biol. 45 (329-347) bzw. aus der EP-A 990892 (PHD 98-123) bekannt. In diesen Veröffentlichungen ist erläutert, daß es einen zentralen, zylinderförmigen Bereich mit ebenen, zur Rotationsachse senkrechten Seitenflächen gibt, innerhalb dessen jedes Voxel von der Strahlenquelle aus einen Winkelbereich von mindestens 180° bestrahlt wird. Außerhalb dieses Bereiches ist eine Rekonstruktion der Schwächung der Strahlung nicht mit hinreichender Qualität möglich. Der zentrale Bereich ist daher der größtmögliche Bereich mit ebenen Seitenflächen, für den ein CT-Bild rekonstruiert werden kann. Dies wird bei der Erfindung ausgenutzt.

In den genannten Veröffentlichungen ist erläutert, daß es in dem zentralen Bereich ein erstes, zur Rotationsachse senkrechtes und sich zu den Seiten kegelförmig verjüngendes Teilvolumen gibt, dessen Voxel in allen Positionen der Strahlenquelle von Strahlung getroffen werden und ein zweites Teilvolumen, dessen Voxel nicht in allen Strahlenquellenpositionen der Strahlung ausgesetzt sind. Bei den bekannten Verfahren werden für jedes zu rekonstruierende Voxel im ersten Teilvolumen gefilterte Daten von Strahlenfächern herangezogen, die das betreffende Voxel aus einem Gesamt-Projektionswinkelbereich von 360° durchstrahlt haben. Demgegenüber werden bei der Erfindung für beide Teile des zentralen Bereiches für jedes Voxel nur gefilterte Daten von Strahlenfächern herangezogen, die das betreffende Voxel zuletzt und aus einem Gesamt-Projektionswinkelbereich von gerade 180° durchstrahlt haben. Der Zeitraum für die Akquisition der zur Rekonstruktion herangezogenen Strahlenfächer ist dabei kürzer als bei dem bekannten Verfahren, und deshalb sind die Artefakte schwächer, die durch eine Bewegung während der Akquisition der Strahlenfächer hervorgerufen werden.

Wie bereits erwähnt, gibt es in dem rekonstruierbaren Bereich ein Teilvolumen, dessen Voxel nicht ständig von einem Strahlenfächer getroffen werden. Ein Teil der Voxel in diesem Teilvolumen wird von allen Strahlenfächern getroffen, die als letzte vor dem Abbildungszeitpunkt in einem Winkelbereich von 180° akquiriert worden sind. Die Rekonstruktion gestaltet sich für diese Voxel analog zu der Rekonstruktion der Voxel, die ständig von Strahlenfächern erfaßt werden. Die anderen Voxel dieses zweiten Teilvolumens werden nicht von allen Strahlenfächern getroffen, die als letzte vor dem Abbildungszeitpunkt in einem Winkelbereich von 180° akquiriert worden sind. Zur Rekonstruktion dieser Voxel müssen daher Strahlenfächer herangezogen werden, die noch weiter zurückliegen, damit sich ein Gesamt-Projektionswinkelbereich der zur Rekonstruktion dieser Voxel herangezogenen Strahlenfächer von 180° ergibt.

In diesen Fällen können Gruppen von Strahlenfächern einander diametral gegenüberliegen. Die Ansprüche 2 und 3 beschreiben alternative Lösungen für die Verarbeitung der gefilterten Daten von diesen Strahlenfächern. Die Lösung nach Anspruch 2 hat das tendenziell bessere Signal/Rauschverhältnis, weil die gefilterten Daten beider Strahlenfächer herangezogen werden, während die Lösung nach Anspruch 3 tendenziell geringere Bewegungsartefakte hat, weil die gefilterten Daten des früher akquirierten Strahlenfächers nicht berücksichtigt werden.

Anspruch 4 beschreibt einen Computertomographen zur Durchführung des Verfahrens nach Anspruch 1, und Anspruch 5 definiert ein Computerprogramm zur Verarbeitung der Meßwerte eines Computertomographen nach Anspruch 4.

Die Erfindung wird nachstehend anhand der Zeichnung näher erläutert. Es zeigen:

Fig. 1 einen Computertomographen zur Durchführung des erfindungsgemäßen Verfahrens,
Fig. 2 ein Ablaufdiagramm dieses Verfahrens,
Fig. 3 ein in einer Strahlenquellenposition erzeugtes kegelförmiges Strahlenbündel,
Fig. 4 eine durch das Rebinning gebildete Gruppe von Strahlenfächern in parallelen Ebenen,
Fig. 5 die geometrischen Verhältnisse im Untersuchungsbereich in einer die Rotationsachse enthaltenden Darstellung und
Fig. 6 die geometrischen Verhältnisse im Untersuchungsbereich in einer dazu senkrechten Darstellung.
Fig. 7 einen bei der Rückprojektion benutzten Speicher

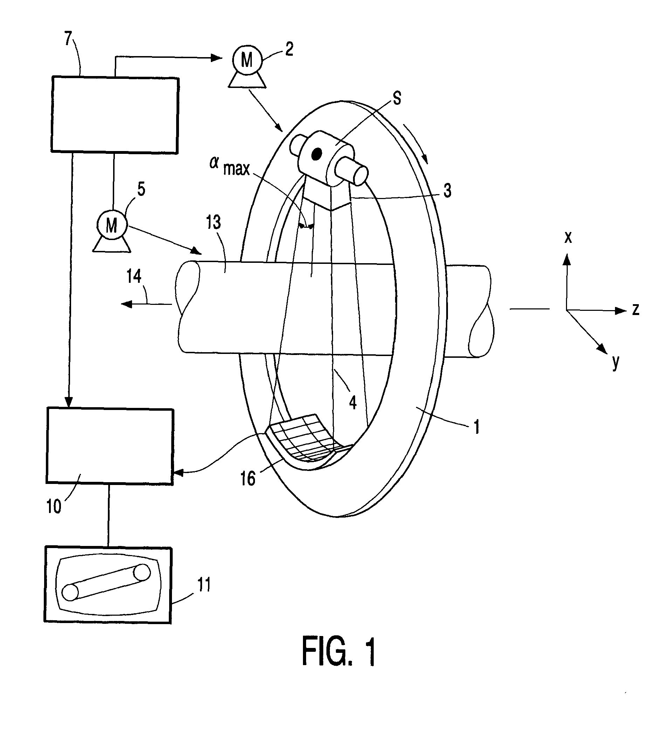

Der in Fig. 1 dargestellte Computertomograph umfaßt eine Gantry 1, die um eine parallel zur z-Richtung des in Fig. 1 dargestellten Koordinatensystems verlaufende Rotationsachse 14 rotieren kann. Dazu wird die Gantry von einem Motor 2 mit einer vorzugsweise konstanten aber einstellbaren Winkelgeschwindigkeit angetrieben. An der Gantry ist eine Strahlenquelle S, beispielsweise ein Röntgenstrahler, befestigt. Dieser ist mit einer Kollimatoranordnung 3 versehen, die aus der von der Strahlenquelle S erzeugten Strahlung ein kegelförmiges Strahlenbündel 4 ausblendet, d. h. ein Strahlenbündel, das sowohl in z-Richtung als auch in einer dazu senkrechten Richtung (d. h. in einer zur Rotationsachse senkrechten Ebene) eine von Null verschiedene endliche Ausdehnung hat.

Das Strahlenbündel durchdringt einen Untersuchungsbereich 13, in dem sich ein Untersuchungsobjekt, z. B. ein Patient auf einem Patientenlagerungstisch (beides nicht näher dargestellt), befinden kann. Der Untersuchungsbereich 13 hat die Form eines Zylinders. Nach dem Durchsetzen des Untersuchungsbereichs 13 trifft das Röntgenstrahlenbündel 4 auf eine an der Gantry 1 befestigte zweidimensionale Detektoreinheit 16. Diese umfaßt eine Anzahl von in z-Richtung nebeneinander angeordneten Detektorzeilen mit jeweils einer Vielzahl von Detektorelementen. Die Detektorzeilen befinden sich in zur Rotationsachse senkrechten Ebene auf einem Kreisbogen um die Strahlenquelle S; sie können aber auch einen Kreisbogen um die Rotationsachse 14 beschreiben oder geradlinig sein. Jedes Detektorelement liefert in jeder Strahlenquellenposition einen Meßwert für einen Strahl aus dem Strahlenbündel 4.

Der mit $\alpha_{max}$ bezeichnete Öffnungswinkel des Strahlenbündels (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse 14 definierten Zentralstrahlebene einschließt) bestimmt den Durchmesser des Zylinders, innerhalb dessen sich das zu untersuchende Objekt bei der Akquisition der Meßwerte befindet. Der Untersuchungsbereich 13 bzw. das Untersuchungsobjekt oder der Patientenlagerungstisch kann mittels eines Motors 5 parallel zur Rotationsachse 14 verschoben werden. Wenn die beiden Motoren 2 und 5 gleichzeitig laufen, ergibt sich eine helixförmige Bewegung der Strahlenquelle S und der Detektoreinheit 16 um den Untersuchungsbereich 13. Wenn hingegen der Motor 5 für den Vorschub in z-Richtung stillsteht und nur der Motor 2 die Gantry einzeln rotieren läßt, ergibt sich eine kreisförmige Abtastbewegung der Strahlenquelle S und der Detektoreinheit 16 relativ zum Untersuchungsbereich 13.

Die von der Detektoreinheit 16 akquirierten Meßdaten werden von einem Bildverarbeitungsrechner 10 zugeführt, der daraus die Absorptionsverteilung in einem Teil des Untersuchungsbereichs 13 rekonstruiert und z. B. auf einem Monitor wiedergibt. Die beiden Motoren 2 und 5, der Bildverarbeitungsrechner 10, die Strahlenquelle S und der Transfer der Meßwerte von der Detektoreinheit 16 zum Bildverarbeitungsrechner 10 werden von einer geeigneten Kontrolleinheit 7 gesteuert.

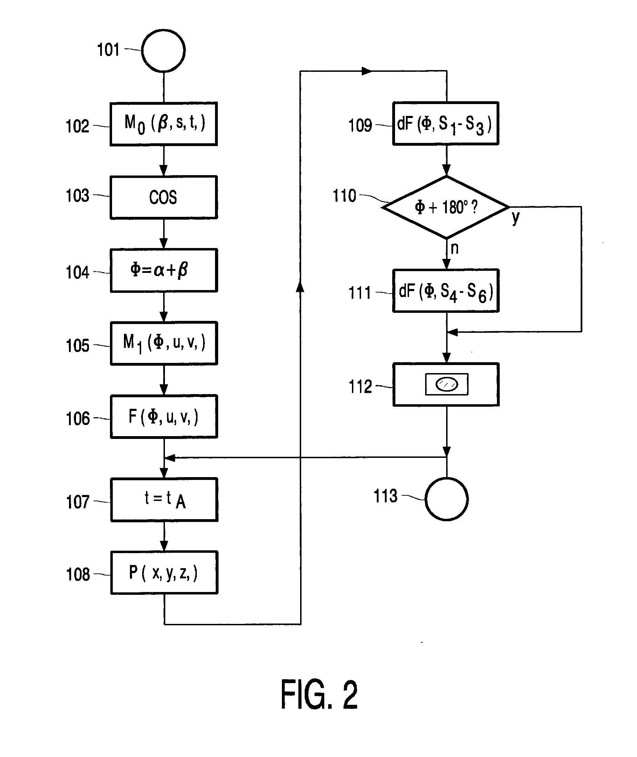

Fig. 2 zeigt den Ablauf eines Meß- und Rekonstruktionsverfahrens, das mit dem Computertomographen nach Fig. 1 durchgeführt werden kann.

Nach der Initialisierung im Block 101 rotiert die Gantry mit einer konstanten Geschwindigkeit, wobei sich eine Umlaufzeit von 1 sec oder weniger ergibt. Die Strahlenquelle S emittiert ein kegelförmiges Strahlenbündel, das den Untersuchungsbereich durchsetzt, und die von den Detektorelementen der Detektoreinheit 16 akquirierten Meßwerte werden im Bildverarbeitungsrechner 10 zwischengespeichert und weiterverarbeitet.

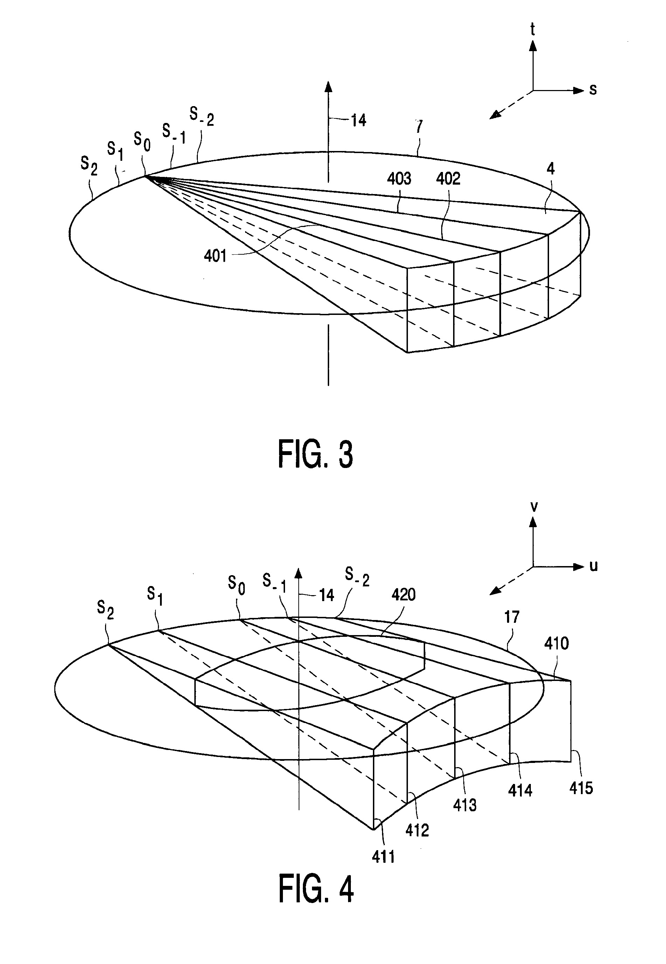

Fig. 3 zeigt die kreisförmige Bahn 17, auf der sich die Strahlenquelle S und die Detektoreinheit 16 um die Rotationsachse 14 bewegen. Für eine bestimmte Strahlenquellenposition $S_0$ ist das Strahlenbündel 4 dargestellt. Dieses kegelförmige Strahlenbündel kann in eine Vielzahl von ebenen Strahlenfächern zerlegt werden, die sich - wie die in Fig. 3 dargestellten Strahlenfächer 401, 402, 403 - in zur Rotationsachse parallelen Ebenen befinden. Die Strahlenfächer gehen von derselben Strahlenquellenposition aus und werden von je einer zur Rotationsrichtung parallelen Spalte von Detektorelementen auf der Detektoreinheit 16 erfaßt.

Fig. 3 deutet an, daß auch in anderen Positionen der Strahlenquelle, z. B. $S_{-2}$, $S_{-1}$, $S_1$ oder $S_2$, das emittierte kegelförmige Strahlenbündel gemessen wird. Diese Strahlenquellenpositionen bzw. die dort emittierten Strahlenbündel (4) können durch einen Parameter $\beta$ charakterisiert werden, der dem Winkel entspricht, den das Lot von der Strahlenquellenposition auf die Rotationsachse 14 mit einer die Rotationsachse enthaltenden Referenzebene einschließt ($\beta$ kann entsprechend der Anzahl der Umdrehungen der Strahlenquelle um die Rotationsachse größer sein als $2\pi$). Die Lage eines jeden Strahlenfächers in einem Strahlenbündel kann durch einen Parameter s gekennzeichnet werden, der die Lage der von dem Strahlenfächer getroffenen Spalte von Detektorelementen innerhalb der Detektoreinheit 16 beschreibt. Jeder Strahl innerhalb eines solchen Strahlenfächers kann wiederum durch den Parameter t charakterisiert werden, der die Lage des von dem betreffenden Strahl getroffenen Detektorelements innerhalb der Spalte von Detektorelementen beschreibt bzw. den Abstand dieses Dektorelements von einer die Kreisbahn 17 enthaltenden zentralen Ebene.

Somit bilden die akquirierten Meßwerte einen dreidimensionalen Datensatz $M_0(\beta, s, t)$, wobei jeder Meßwert einem Gitterpunkte eines regelmäßigen kartesischen Gitters in einem dreidimensionalen ($\beta$, s, t)-Parameterraum entspricht. Die Akquisition der Meßwerte stellt daher eine Abtastung der sogenannten Objektfunktion (in diesem Fall des Linienintegrals der Schwächung der Strahlung) an einer Vielzahl von gleichmäßig im ($\beta$, s, t)- Parameterraum verteilten Punkten dar.

Die Akquisition der Meßwerte im Schritt 102 und die Verarbeitung dieser Meßwerte in den darauf folgenden Schritten 103 ff erfolgen zeitlich parallel zueinander, so daß die vorhandenen Meßwerte bereits weiterverarbeitet werden, während weitere Meßwerte akquiriert werden.

Im Schritt 103 werden die Meßwerte mit dem Cosinus des Winkels multipliziert, den der Strahl, entlang dessen der Meßwert erfaßt wurde, mit einer zur Rotationsachse senkrechten Ebene einschließt. Wenn die Abmessungen der Detektoreinheit in z-Richtung gering sind, kann dieser Schritt aber entfallen, weil der Winkel dann so klein ist, daß der Cosinus des Winkels stets nahezu 1 ist.

Der im Schritt 102 akquirierte und im Schritt 103 gegebenenfalls modifizierte Datensatz $M_0(\beta, s, t)$ ist für die weitere Verarbeitung noch nicht optimal. Deshalb erfolgt in den Schritten 104 und 105 ein sogenanntes Rebinning der Meßwerte. Die Daten werden dabei so umsortiert und uminterpoliert, als wären sie mit einer anderen Strahlenquelle (einer kreisbogenförmigen Strahlenquelle, die zueinander parallele Strahlenfächer emittiert) und mit einem anderen Detektor (einem ebenen, rechteckigen und die Rotationsachse enthaltenden Detektor) gemessen worden. In Schritt 104 werden zu diesem Zweck zunächst die Strahlenfächer von verschiedenen Strahlenquellenpositionen zu jeweils einer Gruppe zusammengefaßt, die in zueinander parallelen Ebenen liegen.

Fig. 4 zeigt eine auf diese Weise gebildete Gruppe von Strahlenfächern. Von jeder Strahlenquellenposition $S_{-2}$ ... $S_0$ ... $S_2$ gehört jeweils ein Strahlenfächer zu dieser Gruppe.

Die zu einer Gruppe gehörenden Strahlenfächer erfüllen die Bedingung

$$\phi = \alpha + \beta \tag{1}$$

Dabei ist $\phi$ der Projektionswinkel, unter dem eine Gruppe von Strahlenfächern den Untersuchungsbereich durchsetzt und den jeder Strahlenfächer dieser Gruppe mit der weiter oben erwähnten, die Rotationsachse enthaltenden Referenzebene einschließt. a ist der Winkel, den der betreffende Strahlenfächer in dem ursprünglichen Strahlenbündel (vgl. Fig. 3) mit einer durch die Rotationsachse 14 und die Strahlenquellenposition (z. B. $S_2$, die ihrerseits durch den Winkel β definiert ist) bestimmten Zentralebene einschließt. Derartige Gruppen von Strahlenfächern werden für verschiedene Projektionswinkel φ gebildet, die sich jeweils um ein bestimmtes Projektionswinkel-Inkrement dφ voneinander unterscheiden. Wenn die Strahlenfächer eines Strahlenbündels die Gleichung 1 nicht exakt erfüllen, muß ein entsprechender Strahlenfächer durch Interpolation aus den Strahlen benachbarter Strahlenfächer des Strahlenbündels (Fig. 3) ermittelt werden.

Die Strahlenfächer einer Gruppe - darunter die in Fig. 4 dargestellten Strahlenfächer 411 ... 415 - definieren ein Strahlenbündel 410, das eine zeltartige Form hat und sich aus Strahlenfächern zusammensetzt, die in zueinander zur Rotationsachse parallelen Ebenen liegen. In Fig. 4 ist außerdem der Schnittbereich 420 dargestellt, der sich ergibt, wenn das Strahlenbündel 410 von einer die Rotationsachse 14 enthaltende und zu den Ebenen der Strahlenfächer 411 ... 415 senkrechten Ebene geschnitten wird. Die oberen und unteren Ränder sind kissenförmig nach außen gewölbt, weil die Strahlenquellenpositionen in der Mitte weiter von der Schnittebene entfernt liegen als die Strahlenquellenpositionen am Rande.

Im Schritt 105 wird nun unter Weglassung der gewölbten oberen und unteren Ränder des Schnittbereichs 420 ein rechteckiger Bereich definiert, und die Strahlen der Strahlenfächer einer Gruppe werden durch Interpolation neu berechnet, und zwar so, daß sie das erwähnte Rechteck im Schnittbereich 420 an den Gitterpunkten eines regelmäßigen kartesischen Gitters durchstoßen, die durch die Koordinaten u (senkrecht zur Rotationsachse) und v (parallel zur Rotationsachse)gekennzeichnet sind. Durch das Rebinning werden also die Meßwerte $M_0(\beta, s, t)$ die durch ein regelmäßiges Gitter im $(\beta, s, t)$-Parameterraum definiert sind, überführt in Meßwerte $M_1(\phi, u, v)$, die durch ein regelmäßiges Gitter in dem dreidimensionalen $(\phi, u, v)$-Parameterraum definiert sind.

Da der Teil der Strahlen des ursprünglichen Strahlenbündels, die den Schnittbereich 420 im gewölbten oberen oder unteren Teil, (d.h. außerhalb des Rechtecks, für das die Meßwerte $M_1(\phi, u, v)$ definiert sind), im weiteren Verfahren nicht mehr benutzt werden, ist es von Vorteil, die Kollimatoranordnung 3 so zu gestalten, daß das kegelförmige Strahlenbündel diese Strahlen nicht enthält. Anstelle von geradlinigen, senkrecht zur Rotationsachse verlaufenden Kanten müßte die Kollimatoranordnung 3 zu diesem Zweck nach innen gewölbte Kanten aufweisen. Dadurch würde die Strahlenbelastung für den Patienten verringert.

Im Schritt 106 erfolgt eine eindimensionale, rampenförmige Filterung der Meßwerte $M_1(\phi, u, v)$. Dabei werden alle Meßwerte mit demselben Projektionswinkel φ und mit dem gleichen Parameter v einer Filterung unterzogen, bei der der Übertragungsfaktor rampenförmig mit der Frequenz ansteigt.

Die auf diese Weise gefilterten Daten $F(\phi, u, v)$ werden anschließend zur Rekonstruktion der Absorptionsverteilung im Untersuchungsbereich durch eine Rückprojektion herangezogen, zunächst aber in einem Zwischenspeicher gespeichert. Während der folgenden Rekostruktionsschritte 107 ff werden die Schritte 103 bis 106 fortlaufend auf die Meßwerte angewandt, die weiterhin (im Schritt 102) akquiriert werden, sodaß sich ein stetiger Strom von auf diese Weise gefilterten Daten $F(\phi, u, v)$ ergibt. Aus diesem Strom gefilterter Daten werden durch je eine Rückprojektion CT Bilder erzeugt, die das Objekt in unterschiedlichen Abtastzeitpunkten darstellen. Um zu vermeiden, daß der Patient einer Strahlenbelastung ausgesetzt wird, ohne daß die dabei erzeugten Meßwerte für die Rekonstruktion benötigt werden, muß der zeitliche Abstand zwischen aufeinanderfolgenden Abbildungszeitpunkten kürzer sein als der Zeitraum, den die Strahlenquelle für eine vollständige Rotation um die Rotationsachse 14 benötigt.

Dementsprechend wird im Schritt 107 ein entsprechender Zeitpunkt $t_A$ vorgegeben. Diesem Abbildungszeitpunkt ist eine bestimmte Strahlenquellenposition (β) zugeordnet. Da zwischen der Akquisition der Meßwerte und der Erzeugung der gefilterten Daten auf diesen Meßwerten im Schritt 106 aber immer eine bestimmte zeitliche Verzögerung besteht, ist die Strahlenquelle inzwischen schon über diese Position hinausgerückt. Die Rückprojektion umfaßt die Vorgabe eines Voxels P(x,y,z) im Schritt 108 und die Summierung der Beiträge der gefilterten Daten von Strahlen, die das betreffende Voxel zuvor getroffen haben, in den Schritten 109 bis 111.

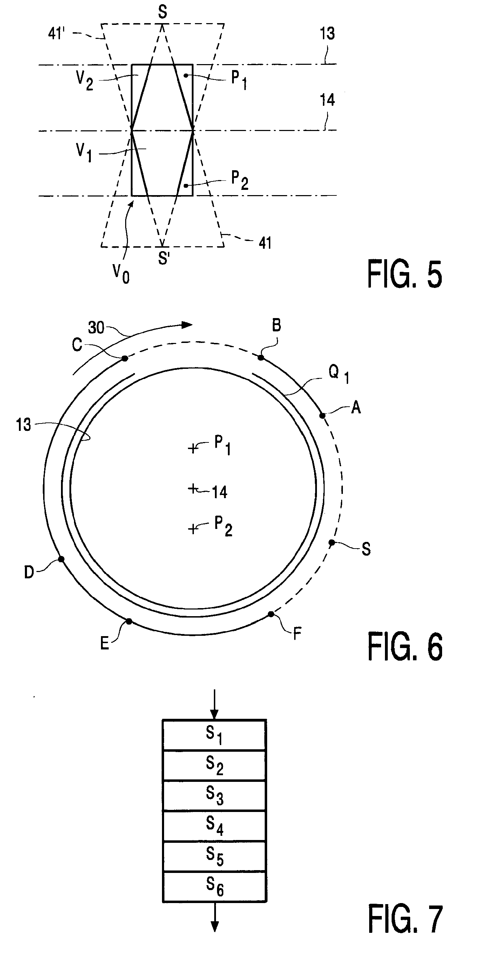

Zur Erläuterung der Rückprojektion wird davon ausgegangen, dass pro Umdrehung der Strahlenquelle sechs CT-Bilder rekonstruiert werden sollen, die das Objekt in je sechs Abbildungs-Zeitpunkten darstellen, denen sechs gleichmäßig auf dem Kreis 17 verteilte Strahlenquellenpostitionen zugeordnet sind. Weiterhin wird vorausgesetzt, dass vor dem Abbildungszeitpunkt die gefilterte Daten über einen Projektions-Winkelbereich vom 360° vorliegen und dass diese in einem Speicher gespeichert sind. Dieser in Fig.7 dargestellte Speicher besitzt eine der Zahl der Abbildungszeitpunkte (im Beispiel sechs) entsprechende Anzahl von Abschnitten $S_1$ bis $S_6$, die Daten für einen Projektionswinkelbereich von je 60° aufnehmen können und die nach Art eines Schieberegisters organisiert sind, bei dem neu eintreffende Daten $F(\phi, u, v)$ die älteren Daten ersetzen. Dementsprechend befinden sich nach dem Schritt 107 im ersten Abschnitt $S_1$ für den Abbildungszeitpunkt $t_A$ die jüngsten Daten $F(\phi, u, v)$ für einen Projektionswinkelbereich von $\phi_A - 60°$ bis $\phi_A$, wobei $\phi_A$ der Projektionswinkel der zuletzt eingegangenen gefilterten Daten $F(\phi_A, u, v)$ ist. Im zweiten Abschnitt $S_2$ befinden sich dann die Daten für einen Projektionswinkelbereich von $\phi_A - 120°$ bis $\phi_A - 60°$, im 3 Abschnitt $S_3$ die Daten für einen Projektionswinkelbereich von $\phi_A - 180°$ bis $\phi_A - 120$, usw.

Im Schritt 109 werden dann die Beiträge $dF(\phi, S_1 - S_3)$ von gefilterten Daten summiert, die in den Abschnitten $S_1$ bis $S_3$ gespeichert sind und die Strahlen zugeordnet sind, die das Voxel aus dem Projektionswinkelbereich von $\phi_A$ bis $\phi_A - 180°$ durchstrahlt haben. Danach wird im Schritt 110 für jeden Projektionswinkel φ ($\phi < \phi_A - 180°$) geprüft, ob das Voxel aus der Projektionsrichtung $\phi+180°$ bestrahlt (und dementsprechend im Schritt 109 ein Beitrag für das Voxel akkumuliert wurde). Wenn dies der Fall war, wird der folgende Schritt 111 übersprungen. Für die anderen Projektionsrichtungen werden im Schritt 109 die Beiträge $dF(\phi,S_4-S_6)$ von gefilterten Daten summiert, die in den Abschnitten $S_4$ bis $S_6$ gespeichert sind und die Strahlen zugeordnet sind, die das Voxel aus dem Projektionswinkelbereich von $\phi_A$ -180° bis $\phi_A$-360° durchstrahlt haben.

Das Ergebnis der Summierung hängt von der Lage des Voxels P(x,y,z) im Untersuchungsbereich ab. Dazu wird auf Fig. 5 und Fig. 6 verwiesen.

Fig. 5 zeigt in gestrichelten Linien einen der Strahlenfächer 41, der durch das Rebinning entstanden ist und der die Rotationsachse 14 durchsetzt. Die Strahlenquelle S befindet sich dabei senkrecht oberhalb der Rotationsachse. Außerdem ist ein Strahlenfächer 41' dargestellt, der aus einer Strahlenquellenposition S' senkrecht unterhalb der Rotationsachse 14 emittiert wird. Die Randstrahlen der beiden Strahlenfächer schneiden sich wegen der Symmetrie der Anordnung in der Rotationsachse 14, und es läßt sich zeigen, daß diese Schnittpunkte die Breite des zylinderförmigen Volumens $V_0$ definieren, in dem die Absorptionsverteilung vollständig rekonstruiert werden kann. Außerhalb dieses Volumens $V_0$ ist eine Rekonstruktion der Absorptionsverteilung im Untersuchungsobjekt 13 nicht möglich.

Das Volumen $V_0$ läßt sich in ein erstes Teilvolumen $V_1$ und ein zweites Teilvolumen $V_2$ unterteilen, die durch die Randstrahlen der Strahlenfächer 41, 41' voneinander getrennt sind. Das erste Teilvolumen $V_1$ im Zentrum ist ein rotationssymmetrischer und diskusförmiger Bereich, während das zweite Teilvolumen $V_2$ alle innerhalb des Volumens $V_0$ beiderseits des ersten Teilvolumens $V_1$ verbleibenden Bereiche umfaßt. Alle Voxel innerhalb des ersten Teilvolumens $V_1$ werden ununterbrochen von Strahlung getroffen. Hingegen werden die Voxel im zweiten Teilvolumen $V_2$ nicht ständig von Strahlung getroffen. Beispielsweise wird das oberhalb der Rotationsachse 14 befindliche Voxel $P_1$ nicht von dem Strahlenfächer 41 getroffen (wohl aber von dem Strahlenfächer 41'), während das genau darunter und symmetrisch zur Rotationsachse 14 angeordnete Voxel $P_2$ von dem Strahlenfächer 41, aber nicht von dem Strahlenfächer 41' getroffen wird.

Es hängt also von der Lage eines Voxels in dem Teilvolumen $V_0$ ab, bei welchen Projektionswinkeln $\phi$ sich im Schritt 109 ein Beitrag $dF(\phi,S_1-S_3)$ für das betreffende Voxel ergibt.

Zur Erläuterung wird auf Fig. 6 verwiesen. Fig. 6 zeigt den Untersuchungsbereich 13 in einem zur Rotationsachse 14 senkrechten Schnitt. Der Kreisbogen $\phi_1$ symbolisiert den Projektionswinkel-Bereich, aus dem das Voxel $P_1$ von Strahlung getroffen werden kann (diese Darstellung vereinfacht die Verhältnisse insofern, als die Strahlenquelle wesentlich weiter von der Rotationsachse entfernt ist als sich aus diesem Bogen ergibt). Alle Voxel im zweiten Teilvolumen haben einen solchen Projektionswinkel-Bereich, der mindestens 180° und weniger als 360° beträgt und der sich in unterschiedlicher Lage bezüglich der Rotationsachse befinden kann.

In Fig. 6 ist noch ein weiterer Kreis teilweise in gestrichelten Linien dargestellt. Die Position der Strahlenquelle im Abbildungszeitpunkt $t_A$ ist mit S bezeichnet. Um den Winkel $2\alpha_{max}$ gegenüber der Position S entgegen der Umlaufrichtung gemäß dem Pfeil 30 versetzt ist die Position A eingezeichnet. Diese Position definiert den Projektionswinkel $\phi_A$, für den aus dem Rebinning der bis zum Abbildungszeitpunkt $t_A$ akquirierten Messwerte gerade noch eine vollständige Gruppe von Strahlenfächern (d. h. eine Gruppe, die den gesamten Querschnitt des Untersuchungsbereichs durchsetzt hat) erzeugt werden kann. Die Positionen B, C, D, E und F bezeichnen Projektionswinkel, $\phi_B$, $\phi_C$, $\phi_D$, $\phi_E$ und $\phi_F$, die aus dem Rebinning früher akquirierter Meßwerte hervorgehen.

Für die Rekonstruktion eines Voxels im ersten Teilvolumen sind die gefilterten Daten von Strahlen erforderlich, die im Projektionswinkelbereich von $\phi_A$ bis $\phi_D$ liegen, also in einem Projektionswinkelbereich von 180°. Das Gleiche gilt für einen Teil der Voxel im zweiten Teilvolumen $V_2$, wenn diese in allen Winkelstellungen $\phi_A...\phi_D$ von einem Strahlenfächer unter einem entsprechenden Projektionswinkel erfaßt worden sind - wie z. B. das Voxel $P_2$. Diese Voxel erhalten in den Schritten 110 und 111 keinen Beitrag mehr von früher akquirierten Daten aus den Abschnitten $S_4$ bis $S_6$ des Speichers.

Bei dem Voxel $P_1$ reicht dieser Projektionswinkelbereich jedoch nicht aus, weil dieses Voxel im Bereich $\phi_B$ bis $\phi_C$ von keinem Strahlenfächer erfaßt wird. Für dieses Voxel müssen die gefilterten Daten von noch früher akquirierten Meßwerten (aus den Abschnitten $S_4$ bis $S_6$ des Speichers) herangezogen werden. Da in der Computertomographie Messungen aus um 180° gegeneinander versetzten Richtungen äquivalent sind, können die fehlenden Daten von Strahlenfächern aus dem Bereich $\phi_B$ bis $\phi_C$ ersetzt werden durch gefilterte Daten der Strahlenfächer aus dem Projektionswinkel-Bereich $\phi_E$ bis $\phi_F$, der dem Projektionswinkel-Bereich $\phi_B$ bis $\phi_C$ diametral gegenüberliegt. Dieses Beispiel zeigt, dass man auf soviel früher akquirierten Messwerte zurückgreifen muß, bis für das jeweilige Voxel ($P_1$) gefilterte Daten aus einem 180° betragenden zusammenhängender Bereich (in diesem Fall $\phi_C$ bis $\phi_F$) von Projektionswinkeln zur Verfügung stehen. Nur dann kann man diesem Voxel Beiträge von Strahlenfächern aus einem Gesamt-Projektionswinkelbereich von gerade 180° zuordnen.

Für das Voxel $P_1$ gibt es in diesem Fall zwei Projektionswinkel-Bereiche - nämlich die Bereiche $\phi_A$-$\phi_B$ und $\phi_D$-$\phi_E$, die sich einander diametral gegenüberliegen. Im Schritt 110 wird das erkannt, und dementsprechend erhält dieses Voxel aus den Abschnitten $S_4$ bis $S_6$ des Speichers keinen Beitrag für den Projektionswinkelbereiche $\phi_D$-$\phi_E$. Jedoch ergibt sich eine Beitrag aus den Abschnitten $S_4$ bis $S_6$ für den Bereich $\phi_E$-$\phi_F$, weil das Voxel aus dem Bereich $\phi_B$ bis $\phi_C$ keinen Beitrag erhalten hat.

Es gibt aber noch eine andere Möglichkeiten, die gefilterten Daten aus diesen Bereichen zu verarbeiten. Man kann nämlich für das Voxel $P_1$ Beiträge sowohl vom Projektionswinkel-Bereich $\phi_A$-$\phi_B$ als auch von dem Bereich $\phi_D$-$\phi_E$ mit zwei Gewichtungsfaktoren summieren (z. B. 0,5), deren Summe genauso groß ist wie das Gewicht, mit dem die gefilterten Daten von Strahlenfächern aus dem Bereich $\phi_C$-$\phi_D$ oder $\phi_E$-$\phi_F$ bei der Rückprojektion berücksichtigt werden.

Das auf diese Weise rekonstruierte CT-Bild der Absorption der Strahlung im Teilvolumen $V_0$ wird auf geeignete Weise auf dem Monitor 11 wiedergegeben (Schritt 112). Währenddessen werden die Schritte 103 bis 106 fortlaufend auf die Meßwerte angewandt, die weiterhin (im Schritt 102) akquiriert werden. Aus dem daraus resultierenden Strom gefilterter Daten wird für einen späteren Abbildungszeitpunkt ein weiteres CT-Bild rekonstruiert, dem eine z. B. um 60° in Umlaufrichtung vorgerückte Strahlenquellenposition zugeordnet ist.

Es werden dann die zuletzt erzeugten Daten gefilterten Daten F($\phi$,u,v) für einen Projektionswinkelbereich von 60° in den ersten Abschnitt $S_1$ des Speichers übernommen, wobei die zuvor dort gespeicherten Daten einen Abschnitt weiterrücken. Die ältesten, zuvor im letzten Abschnitt $S_6$ gespeicherten Daten werden gelöscht. Die Schleife mit den Schritten 107 bis 112 wird dann so oft durchlaufen, bis die CT-Fluoroskopie im Schritt 113 vom Benutzer beendet wird.

Vorstehend wurde davon ausgegangen, dass sich die Strahlenquelle mit der Detektoreinheit um die Rotationsachse 14 dreht, während der Untersuchungsbereich bzw. das darin befindliche Untersuchungsobjekt ruht. Dies ist bei medizinische Untersuchungen notwendig. Bei industriellen Untersuchungen von z.B. Werkstücken hingegen, kann das Untersuchungsobjekt rotieren und können die Strahlenquelle und die Detektoreinheit ruhen. Die Erfindung ist auch in diesem Fall anwendbar. Der Projektionswinkel $\phi$ und die Strahlenquellenposition $\beta$ sind dabei allerdings durch die Winkelstellung des Untersuchungsobjektes definiert.

## Patentansprüche

1. Fluoroskopisches Computertomographie-Verfahren mit den Schritten:

   - Fortlaufende Durchstrahlung eines Untersuchungsobjekts mit einem von einer Strahlenquelle emittierten kegelförmigen Strahlenbündel, wobei das Untersuchungsobjekt oder die Strahlenquelle um eine Rotationsachse rotieren,
   - fortlaufende Akquisition von Meßwerten, die von der Intensität in dem Strahlenbündel jenseits des Untersuchungsobjektes abhängen, mit einer Detektoreinheit,
   - fortlaufende Rekonstruktion von CT-Bildern, die das Untersuchungsobjekt in Abbildungszeitpunkten darstellen, deren zeitlicher Abstand voneinander kürzer ist als der für eine Rotation des Untersuchungsobjektes oder der Strahlenquelle um die Rotationachse benötigte Zeitraum, wobei zu rekonstruierende Voxel solche Voxel beinhalten, die nicht ständig von Strahlung getroffen werden, wobei die Rekonstruktion folgende Schritte umfasst:

   a) Rebinning der Meßwerte zu einer Anzahl von Gruppen, wobei jede Gruppe die Messwerte von bei der Durchstrahlung erzeugten Strahlenfächern umfasst, die in zueinander und zur Rotationsachse parallelen Ebenen liegen,
   b) eindimensionale Filterung der durch das Rebinning erzeugten Daten einer jeden Gruppe zur Erzeugung gefilterter Daten,
   c) Rückprojektion der gefilterten Daten von mehreren Gruppen, wobei für jedes zu rekonstruierende Voxel gefilterte Daten aus den letzten vor dem jeweiligen Abbildungszeitpunkt erzeugten Strahlenfächern herangezogen werden, die das betreffende Voxel aus einem Gesamt-Projektionswinkelbereich von gerade 180° durchstrahlt haben.

2. Fluoroskopisches Computertomographie-Verfahren nach Anspruch 1, wobei zur Rekonstruktion der Absorption in nur zeitweise im Strahlenbündel befindlichen Voxeln die gefilterten Daten von Strahlenfächern, die das Voxel aus um 180° gegeneinander versetzten Projektionswinkel-Bereichen ($\phi_A$-$\phi_B$ bzw. $\phi_D$-$\phi_E$) durchstrahlt haben und sich innerhalb des Gesamt-Projektionswinkelbereichs von 180° befinden, gewichtet summiert werden.

3. Fluoroskopisches Computertomographie-Verfahren nach Anspruch 1, wobei zur Rekonstruktion der Absorption in nur zeitweise im Strahlenbündel befindlichen Voxeln von den gefilterten Daten der Strahlenfächer, die das Voxel aus um 180° gegeneinander versetzten Richtungen durchstrahlt haben und sich innerhalb des Gesamt-Projektionswinkelbereichs von 180° befinden, nur Daten ($\phi_A$-$\phi_B$) des zuletzt erzeugten Strahlenfächers herangezogen werden.

4. Computertomograph zur Durchführung des Verfahrens nach Anspruch 1

- mit einer Strahlenquelle (S) zur fortlaufenden Erzeugung eines kegelförmigen Strahlenbündels (4),
- mit einer Antriebs-Anordnung (2,5) zur Erzeugung einer kreisförmigen Relativbewegung zwischen der Strahlenquelle (S) und dem Untersuchungsobjekt,
- mit einer Detektoreinheit (16) zur fortlaufenden Akquisition von Meßwerten, die von der Schwächung der Strahlen in dem Untersuchungsbereich abhängen, und
- mit einer Rekonstruktionseinheit zur fortlaufenden Rekonstruktion von CT-Bildern, die das Untersuchungsobjekt in Abbildungszeitpunkten darstellen, deren zeitlicher Abstand voneinander kürzer ist als der für eine Rotation des Untersuchungsobjektes oder der Strahlenquelle um die Rotationachse benötigte Zeitraum, wobei zu rekonstruierende Voxel solche Voxel beinhalten, die nicht ständig von Strahlung getroffen werden, die die Meßwerte entsprechend folgendem Ablauf verarbeitet:

a) Rebinning der Meßwerte zu einer Anzahl von Gruppen, wobei jede Gruppe die Messwerte von bei der Durchstrahlung erzeugten Strahlenfächern umfasst, die in zueinander und zur Rotationsachse parallelen Ebenen liegen,
b) eindimensionale Filterung der durch das Rebinning erzeugten Daten einer jeden Gruppe zur Erzeugung gefilterter Daten,
c) Rückprojektion der gefilterten Daten von mehreren Gruppen, wobei für jedes zu rekonstruierende Voxel gefilterte Daten aus den letzten vor dem jeweiligen Abbildungszeitpunkt erzeugten Strahlenfächern herangezogen werden, die das betreffende Voxel aus einem Gesamt-Projektionswinkelbereich von gerade 180° durchstrahlt haben.

5. Computerprogramm zur Verarbeitung der Meßwerte eines Computertomographen nach Anspruch 4 mit den Schritten:

a) Rebinning der Meßwerte zu einer Anzahl von Gruppen, wobei jede Gruppe die Messwerte von bei der Durchstrahlung erzeugten Strahlenfächern umfasst, die in zueinander und zur Rotationsachse parallelen Ebenen liegen,
b) eindimensionale Filterung der durch das Rebinning erzeugten Daten einer jeden Gruppe zur Erzeugung gefilterter Daten,
c) Rückprojektion der gefilterten Daten von mehreren Gruppen, wobei für jedes zu rekonstruierende Voxel gefilterte Daten aus den letzten vor dem jeweiligen Abbildungszeitpunkt erzeugten Strahlenfächern herangezogen werden, die das betreffende Voxel aus einem Gesamt-Projektionswinkelbereich von gerade 180° durchstrahlt haben.

**Claims**

1. Fluoroscopic computer tomography method having the steps:

- continuous radiography of a subject of investigation with one cone-shaped beam of rays emitted from a radiation source, wherein the subject of investigation or the radiation source rotate about an axis of rotation,
- continuous acquisition of measured values, which depend on the intensity within the beam of rays that are beyond the subject of investigation, by means of a detector unit,
- continuous reconstruction of CT images, which represent the subject of investigation at different points in time during imaging, the temporal interval of each said CT image being shorter than the time period required for one rotation of the subject of investigation or of the radiation source about the axis of rotation, wherein voxels to be reconstructed contain those voxels that are not continuously hit by radiation, the reconstruction comprising the following steps:

(a) rebinning the measured values into a number of groups, each group comprising the measured values of the beam fans generated during radiography, which beam fans lie in planes parallel to one another as well as to the axis of rotation,
(b) one-dimensional filtering of the data, which were generated by rebinning, of each group in order to generate filtered data,
(c) rear projection of the filtered data of a plurality of groups, wherein for each voxel to be reconstructed, filtered data are used from the last-generated beam fans prior to the respective moment of imaging, which beam fans have penetrated by radiation the voxel concerned from an entire projection angle range of precisely 180°.

**EP 1 310 785 B1**

2. Fluoroscopic computer tomography method as set forth in claim 1, wherein to reconstruct the absorption into voxels that are found only temporarily in the beam of rays, the filtered data from beam fans that have penetrated by radiation the voxel from projection angle ranges ($\phi_A$-$\phi_B$ or $\phi_D$-$\phi_E$) that are offset from one another by 180° and that are also located within the entire projection angle range of 180°, are summed in a weighted manner.

3. Fluoroscopic computer tomography method as set forth in claim 1, wherein to reconstruct the absorption into voxels that are found only temporarily in the beam of rays from the filtered data of the beam fans, which beam fans penetrated by radiation the voxels from directions offset from one another by 180° and which are also located within the entire projection angle range of 180°, only data ($\phi_A$-$\phi_B$) from the most recently generated beam fan are used.

4. Computed tomograph for carrying out the method as set forth in claim 1

   - having a radiation source (S) for continuous generation of a cone-shaped beam of rays (4),
   - having a drive arrangement (2, 5) for generating a circular relative movement between the radiation source (S) and the subject of investigation,
   - having a detector unit (16) for continuous acquisition of measured values that are dependent on the beam attenuation in the investigation range, and
   - having a reconstruction unit for continuous reconstruction of CT images, which represent the subject of investigation at the moments of imaging, the temporal interval of each said CT image being shorter than the time period required for one rotation of the subject of investigation or of the radiation source about the axis of rotation, wherein voxels to be reconstructed contain those voxels that are not continuously hit by radiation and that process the measured values corresponding to the following sequence:

      (a) rebinning the measured values into a number of groups, each group comprising the measured values of the beam fans generated during radiography, which beam fans lie in planes parallel to one another as well as to the axis of rotation,
      (b) one-dimensional filtering of the data, which were generated by rebinning, of each group in order to generate filtered data,
      (c) rear projection of the filtered data of a plurality of groups, wherein for each voxel to be reconstructed, filtered data are used from the last-generated beam fans prior to the respective moment of imaging, which beam fans have penetrated by radiation the voxel concerned from an entire projection angle range of precisely 180°.

5. Computer program for processing the measured values of a computer tomograph as set forth in claim 4 having the following steps:

      (a) rebinning the measured values into a number of groups, each group comprising the measured values of the beam fans generated during radiography, which beam fans lie in planes parallel to one another as well as to the axis of rotation,
      (b) one-dimensional filtering of the data, which were generated by rebinning, of each group in order to generate filtered data,
      (c) rear projection of the filtered data of a plurality of groups, wherein for each voxel to be reconstructed, filtered data are used from the last-generated beam fans prior to the respective moment of imaging, which beam fans have penetrated by radiation the voxel concerned from an entire projection angle range of precisely 180°.

**Revendications**

1. Procédé de tomographie de fluorescence assistée par ordinateur comprenant les étapes de :

      - irradiation continue d'un objet d'examen par un faisceau de rayons de forme conique émis par une source de rayonnement, l'objet d'examen ou la source de rayonnement tournant autour d'un axe de rotation,
      - acquisition continue de valeurs de mesure, qui dépendent de l'intensité dans le faisceau de rayons au-delà de l'objet d'examen, avec une unité de détection,
      - reconstruction continue d'images de tomographie assistée par ordinateur qui représentent l'objet d'examen à des moments de prise d'image, dont l'intervalle temporel est inférieur au laps de temps nécessaire à une rotation de l'objet d'examen ou de la source de rayonnement autour de l'axe de rotation, dans lequel des voxels à reconstruire contiennent des voxels qui ne sont pas irradiés en permanence par le rayonnement, la reconstruction comprenant les étapes suivantes :

a) réarrangement des valeurs de mesure en un certain nombre de groupes, chaque groupe comprenant les valeurs de mesure d'éventails de rayons produits lors de l'irradiation et situés dans des plans parallèles les uns aux autres et parallèles à l'axe de rotation,
b) filtrage unidimensionnel des données générées par le réarrangement de chaque groupe pour la production de données filtrées,
c) rétroprojection des données filtrées de plusieurs groupes, des données filtrées des derniers éventails de rayons produits avant le moment de prise d'image respectif qui ont irradié le voxel concerné à partir d'une plage d'angle de projection totale de juste 180° étant utilisées pour chaque voxel à reconstruire.

**2.** Procédé de tomographie de fluorescence assistée par ordinateur selon la revendication 1, dans lequel les données filtrées d'éventails de rayons, qui ont irradié le voxel à partir de plages d'angle de projection ($\phi_A$-$\phi_B$ et $\phi_D$-$\phi_E$) décalées les unes par rapport aux autres de 180° et qui se trouvent dans la plage d'angle de projection totale de 180°, sont additionnées de manière pondérée pour la reconstruction de l'absorption dans des voxels situés que temporairement dans le faisceau de rayons.

**3.** Procédé de tomographie de fluorescence assistée par ordinateur selon la revendication 1, dans lequel des données filtrées des éventails de rayons qui ont irradié le voxel à partir de directions décalées les unes par rapport aux autres de 180° et qui se trouvent dans la plage d'angle de projection totale de 180°, seules les données ($\phi p_A$-$\phi_B$) du dernier éventail de rayons produit sont utilisées pour la reconstruction de l'absorption dans des voxels situés que temporairement dans le faisceau de rayons.

**4.** Tomographe assisté par ordinateur pour la réalisation du procédé selon la revendication 1

- avec une source de rayonnement (S) pour la production continue d'un faisceau de rayons de forme conique (4),
- avec un agencement d'entraînement (2, 5) pour la génération d'un mouvement circulaire relatif entre la source de rayonnement (S) et l'objet d'examen,
- avec une unité de détection (16) pour l'acquisition continue de valeurs de mesure, qui dépendent de l'atténuation des rayons dans la zone d'examen, et
- avec une unité de reconstruction pour la reconstruction continue d'images de tomographie assistée par ordinateur qui représentent l'objet d'examen à des moments de prise d'image, dont l'intervalle temporel est inférieur au laps de temps nécessaire à une rotation de l'objet d'examen ou de la source de rayonnement autour de l'axe de rotation, dans lequel des voxels à reconstruire contiennent des voxels qui ne sont pas irradiés en permanence par le rayonnement, laquelle traite les valeurs de mesure conformément au déroulement suivant :

a) réarrangement des valeurs de mesure en un certain nombre de groupes, chaque groupe comprenant les valeurs de mesure d'éventails de rayons produits lors de l'irradiation et situés dans des plans parallèles les uns aux autres et parallèles à l'axe de rotation,
b) filtrage unidimensionnel des données générées par le réarrangement de chaque groupe pour la production de données filtrées,
c) rétroprojection des données filtrées de plusieurs groupes, des données filtrées des derniers éventails de rayons produits avant le moment de prise d'image respectif qui ont irradié le voxel concerné à partir d'une plage d'angle de projection totale de juste 180° étant utilisées pour chaque voxel à reconstruire.

**5.** Programme informatique pour le traitement des valeurs mesurées par un tomographe assisté par ordinateur selon la revendication 4 comprenant les étapes de :

a) réarrangement des valeurs de mesure en un certain nombre de groupes, chaque groupe comprenant les valeurs de mesure d'éventails de rayons produits lors de l'irradiation et situés dans des plans parallèles les uns aux autres et parallèles à l'axe de rotation,
b) filtrage unidimensionnel des données générées par le réarrangement de chaque groupe pour la production de données filtrées,
c) rétroprojection des données filtrées de plusieurs groupes, des données filtrées des derniers éventails de rayons produits avant le moment de prise d'image respectif qui ont irradié le voxel concerné à partir d'une plage d'angle de projection totale de juste 180° étant utilisées pour chaque voxel à reconstruire.

FIG. 1

**FIG. 2**

FIG. 3

FIG. 4

FIG. 5

FIG. 6

FIG. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 948930 A
- EP 919954 A
- EP 989520 A
- EP 990892 A

### In der Beschreibung aufgeführte Nicht-Patentliteratur

- **AUFSATZ VON GRASS et al.** *Phys. med. Biol.,* vol. 45, 329-347